# EUROPEAN PATENT APPLICATION

(11) **EP 2 770 093 A1**
(43) Date of publication of application: **27.08.2014**
(21) Application number: 13156755.4
(22) Date of filing: 26.02.2013
(51) Int. Cl.: D02G 3/44, D03D 1/00

(54) **Moisture-retaining and electrically conductive structure**

(71) Applicant: King's Metal Fiber Technologies Co., Ltd., Taichung City (TW)
(72) Inventor: Huang, Hong-Hsu, Taipei City (TW); Yang, Shun-Tung, Taipei City (TW); Peng, Jung-Hsiang, Taipei City (TW); Su, I-Chen, Taipei City (TW)
(74) Representative: Viering, Jentschura & Partner Patent- und Rechtsanwälte

(57) **Abstract**

A moisture-retaining and electrically conductive structure includes at least one electrically conductive textile unit and at least one moisture-retaining textile unit, wherein the electrically conductive textile unit and the moisture-retaining textile unit are interlaced and woven together to form a planar structure. With such a structure, fabric of the same layer can provide both functions of moisture retention and electrical conduction and may improve the lifespan and electrical conductivity.

## Description

### FIELD OF THE INVENTION

The present invention relates to a moisture-retaining and electrically conductive structure, and in particular to a moisture-retaining and electrically conductive structure that provides both functions of moisture retention and electrical conduction.

### BACKGROUND OF THE INVENTION

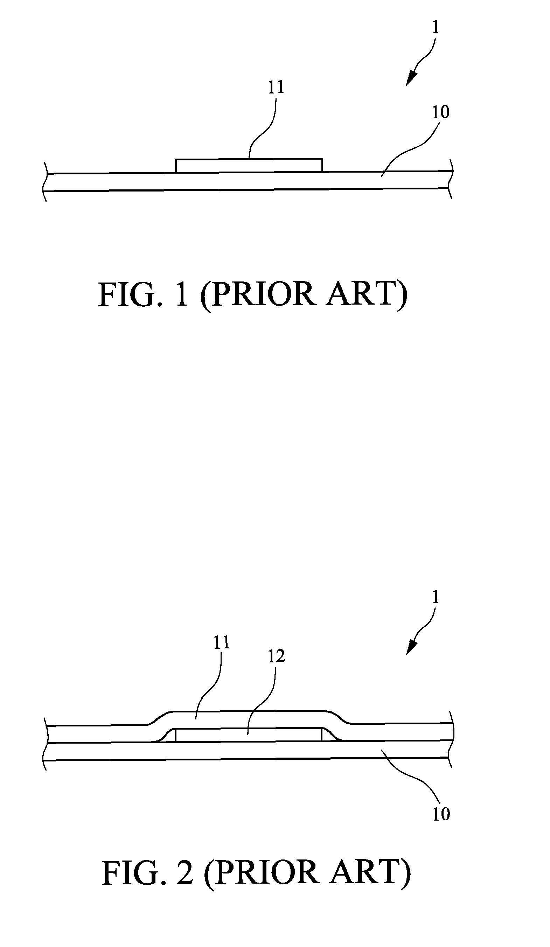

As shown in Figure 1, a conventional detection element 1 for physiological examination comprises a base layer 10 and an electrically conductive layer 11 formed on the base layer 10. To use, the electrically conductive layer is attached to human skin surface to detect a signal generated by the human body. However, in certain areas where the humidity is extremely low, electrical conduction between the electrically conductive layer 11 of the detection element 1 and the human skin is poor, making it difficult to detect the signal generated by the human body. As shown in Figure 2, an improvement is made such that a moisture-retaining layer 12 is arranged between the electrically conductive layer 11 and the base layer 10, wherein the moisture-retaining layer 12 functions to absorb and keep liquid therein in order to increase the humidity between the electrically conductive layer 11 and human skin and thus improve electrical conductivity of the electrically conductive layer 11. However, since the moisture-retaining layer 12 and the electrically conductive layer 11 are two separate layers, moisture must penetrate through the electrically conductive layer 11 before being absorbed by the moisture-retaining layer 12. Consequently, the absorbability of moisture is affected. When the moisture-retaining layer 12 releases water between the electrically conductive layer 11 and human skin, the release of water is also affected by being blocked by the electrically conductive layer 11. Further, since the moisture-retaining layer 12 and the electrically conductive layer 11 are two separate layers that are bonded to each other by an external force (such as adhesion). These layers are easily detached from each other due to the high humidity long maintained by the moisture-retaining layer 12, making the detection element 1 losing its function.

In view of this problem, the present invention aims to provide a structure that provides both functions of moisture retention and electrical conduction in order to achieve the goal of improving electrical conduction and lifespan of product.

### SUMMARY OF THE INVENTION

An object of the present invention is to provide a moisture-retaining and electrically conductive structure that provides both functions of moisture retention and electrical conduction.

To realize the above objectives, the present invention provides a moisture-retaining and electrically conductive structure, which comprises at least one electrically conductive textile unit and at least one moisture-retaining textile unit, wherein the electrically conductive textile unit and the moisture-retaining textile unit are interlaced and woven together to form a planar structure.

In the moisture-retaining and electrically conductive structure discussed above, the electrically conductive textile unit is one of metal fiber, carbon nanotube fiber, and carbon fiber.

In the moisture-retaining and electrically conductive structure discussed above, the electrically conductive textile unit is one of metal fiber yarn, carbon nanotube fiber yarn, and carbon fiber yarn.

In the moisture-retaining and electrically conductive structure discussed above, the moisture-retaining textile unit is one of porous fiber, alginate fiber, carboxymethyl cellulose fiber, and rayon fiber.

In the moisture-retaining and electrically conductive structure discussed above, the moisture-retaining textile unit is one of porous fiber yarn, alginate fiber yarn, carboxymethyl cellulose fiber yarn, and rayon fiber yarn.

In the moisture-retaining and electrically conductive structure discussed above, the electrically conductive textile unit and the moisture-retaining textile unit are woven through plain weaving to form the planar structure.

In the moisture-retaining and electrically conductive structure discussed above, the electrically conductive textile unit and the moisture-retaining textile unit are woven through knitting to form the planar structure.

In the moisture-retaining and electrically conductive structure discussed above, the electrically conductive textile unit and the moisture-retaining textile unit are processed in a non-woven manner to form the planar structure.

### BRIEF DESCRIPTION OF THE DRAWINGS

The present invention will be apparent to those skilled in the art by reading the following description of preferred embodiments thereof with reference to the drawings, in which:

Figure 1 is a side elevational view showing a conventional detection element for physiological examination;

Figure 2 is a side elevational view showing a conventional detection element for physiological examination;

Figure 3 is a schematic view showing a moisture-retaining and electrically conductive structure according to a first embodiment of the present invention;

Figure 4 is a schematic view showing a moisture-retaining and electrically conductive structure according to a second embodiment of the present invention; and

Figure 5 is a schematic view showing a moisture-retaining and electrically conductive structure according to a third embodiment of the present invention.

### DETAILED DESCRIPTION OF THE PREFERRED EMBODIMENTS

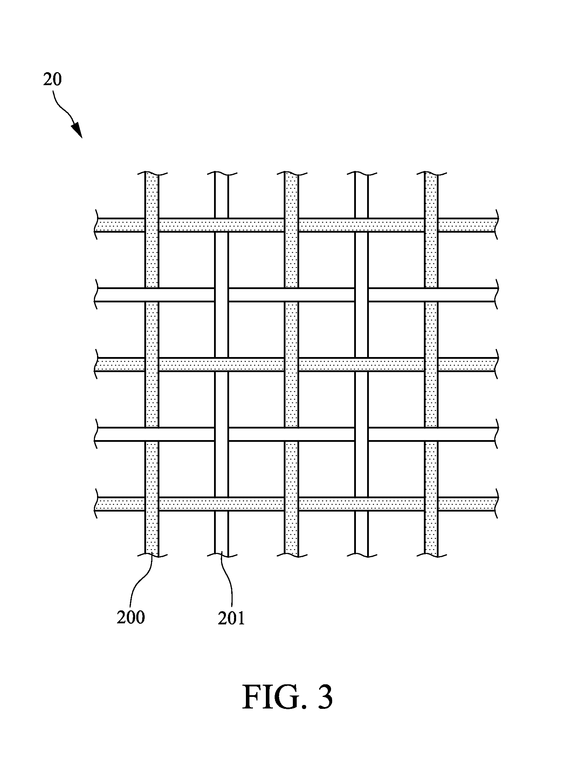

With reference to the drawings and in particular to Figure 3, which is a schematic view showing a moisture-retaining and electrically conductive structure according to a first embodiment of the present invention, as shown in the drawings, in the instant embodiment, the moisture-retaining and electrically conductive structure 20 according to the present invention comprises at least one electrically conductive textile unit 200 and at least one moisture-retaining textile unit 201, both being of a large number in the instant embodiment. These electrically conductive textile units 200 and moisture-retaining textile units 201 are interlaced and woven to form a planar structure by means of plain weaving.

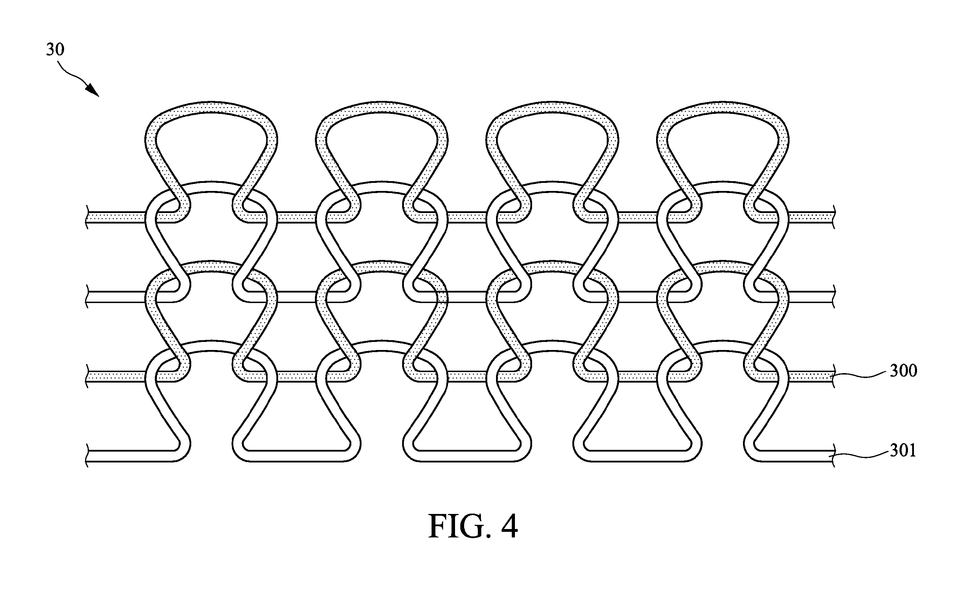

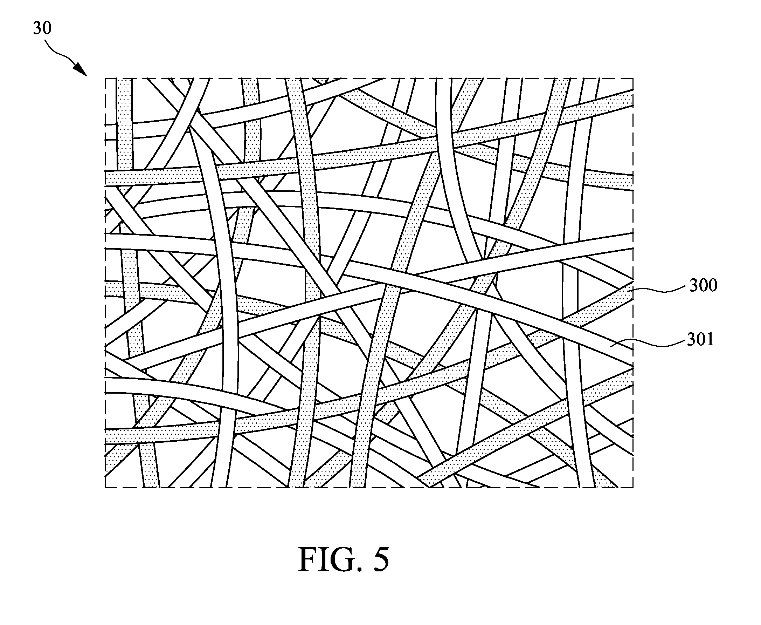

Referring to Figure 4, which is a schematic view showing a moisture-retaining and electrically conductive structure according to a second embodiment of the present invention, as shown in the drawing, in the instant embodiment, the moisture-retaining and electrically conductive structure 30 comprises a plurality of electrically conductive textile units 300 and a plurality of moisture-retaining textile units 301, which are knitted to form a planar structure by means of knitting. The electrically conductive textile unit 300 can be metal fiber yarn, carbon nanotube fiber yarn, and carbon fiber yarn or one metal fiber, carbon nanotube fiber, and carbon fiber. The moisture-retaining textile unit 201 can be porous fiber yarn, alginate fiber yarn, carboxymethyl cellulose fiber yarn, and rayon fiber yarn or one of porous fiber, alginate fiber, carboxymethyl cellulose fiber, and rayon fiber. Further, referring to Figure 5, the moisture-retaining and electrically conductive structure according to the present invention can alternatively be manufactured through a non-woven manner, whereby the electrically conductive textile units 300 and the moisture-retaining textile units 301 are set in an irregularly interlacing fashion.

The moisture-retaining and electrically conductive structure as discussed above are formed as a planar structure through weaving the electrically conductive textile units and the moisture-retaining textile units by means of plain weaving, knitting, or non-woven manner in order to allow the moisture-retaining textile units to directly absorb or release water to the space between the moisture-retaining and electrically conductive unit and human skin. Further, the electrically conductive textile units and the moisture-retaining textile units are closely set in the same layer and the influence on the functionality due to the electrically conductive textile units and the moisture-retaining textile units are separated from each other by the moisture-retaining and electrically conductive unit being long kept humid. The present invention can effectively improve the shortcoming of the conventional device.

Although the present invention has been described with reference to the preferred embodiments thereof, it is apparent to those skilled in the art that a variety of modifications and changes may be made without departing from the scope of the present invention which is intended to be defined by the appended claims.

## Claims

1. A moisture-retaining and electrically conductive structure, comprising:
at least one electrically conductive textile unit; and
at least one moisture-retaining textile unit;
wherein the electrically conductive textile unit and the moisture-retaining textile unit are interlaced and woven together to form a planar structure.

2. The moisture-retaining and electrically conductive structure as claimed in Claim **1,** wherein the electrically conductive textile unit is one of metal fiber, carbon nanotube fiber, and carbon fiber.

3. The moisture-retaining and electrically conductive structure as claimed in Claim **1,** wherein the electrically conductive textile unit is one of metal fiber yarn, carbon nanotube fiber yarn, and carbon fiber yarn.

4. The moisture-retaining and electrically conductive structure as claimed in Claim **1,** wherein the moisture-retaining textile unit is one of porous fiber, alginate fiber, carboxymethyl cellulose fiber, and rayon fiber.

5. The moisture-retaining and electrically conductive structure as claimed in Claim **1,** wherein the moisture-retaining textile unit is one of porous fiber yarn, alginate fiber yarn, carboxymethyl cellulose fiber yarn, and rayon fiber yarn.

6. The moisture-retaining and electrically conductive structure as claimed in Claim **1,** wherein the electrically conductive textile unit and the moisture-retaining textile unit are woven through plain weaving to form the planar structure.

7. The moisture-retaining and electrically conductive structure as claimed in Claim **1,** wherein the electrically conductive textile unit and the moisture-retaining textile unit are woven through knitting to form the planar structure.

8. The moisture-retaining and electrically conductive structure as claimed in Claim **1,** wherein the electrically conductive textile unit and the moisture-retaining textile unit are processed in a non-woven manner to form the planar structure.
